(19) Europäisches Patentamt / European Patent Office / Office européen des brevets

(11) **EP 2 166 838 B1**

(12) # EUROPEAN PATENT SPECIFICATION

(45) Date of publication and mention of the grant of the patent:
**24.10.2018 Bulletin 2018/43**

(51) Int Cl.:
*A61K 8/35* (2006.01)  *A61K 8/43* (2006.01)
*A61Q 11/00* (2006.01)  *A61K 8/9789* (2017.01)

(21) Application number: **08794338.7**

(22) Date of filing: **02.06.2008**

(86) International application number:
**PCT/US2008/007028**

(87) International publication number:
**WO 2008/156559 (24.12.2008 Gazette 2008/52)**

(54) **TOPICAL DENTAL SOLUTION OF CHLORHEXIDINE IN SUMATRA BENZOIN BP/EP AND METHODS OF MANUFACTURING AND EVALUATING SAME**

TOPISCHE ZAHNÄRZTLICHE LÖSUNG AUS CHLORHEXIDIN IN SUMATRA-BENZOIN BP/EP UND VERFAHREN ZU IHRER HERSTELLUNG UND BEWERTUNG

SOLUTION DENTAIRE TOPIQUE DE CHLORHEXIDINE DANS DU BENJOIN BP/EP ET PROCÉDÉS DE FABRICATION ET D'ÉVALUATION DE CELLE-CI

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **01.06.2007 US 932913 P**

(43) Date of publication of application:
**31.03.2010 Bulletin 2010/13**

(73) Proprietor: **CHX Technologies Inc.**
**Toronto,**
**Ontario M9A 1B1 (CA)**

(72) Inventors:
• **PERRY, Oliver, Ross**
**Toronto, ON M9C 3C3 (CA)**
• **TURGEON, Brenda**
**Oakville, ON L6L 3C5 (CA)**
• **SYMINGTON, John, M.**
**Etobicoke, ON M9C 3J8 (CA)**

(74) Representative: **Jacob, Reuben Ellis**
**Maucher Jenkins**
**26 Caxton Street**
**London SW1H 0RJ (GB)**

(56) References cited:
WO-A1-98/35709    US-A- 4 474 807
US-A- 4 496 322    US-A- 4 496 322
US-A- 4 883 534    US-A- 5 178 870
US-A1- 2003 162 839    US-A1- 2006 110 379
US-A1- 2006 228 416    US-A1- 2006 270 637

• **Raul Marino Jr ET AL: "Multiple use of benzoin as an aid in neurosurgical practice the watertight benzoin dressing Usos do benjoim na prática neurocirúrgica", Arquivos de Neuro-Psiquiatria, 1 December 1979 (1979-12-01), pages 373-379, XP055160028, DOI: 10.1590/S0004-282X1979000400003 Retrieved from the Internet: URL:http://doaj.org/search?source=%7B%22query%22%3A%7B%22bool%22%3A%7B%22must%22%3A% 5B%7B%22term%22%3A%7B%22id%22%3A%2 236af6f3 9cf58473a80ee95c15cec13d7%22%7D%7D%5D %7D%7 D%7D**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Background of the Invention**

**FIELD OF THE INVENTION**

A topical, antibacterial coating according to claim 6 and methods of manufacturing according to claim 1 as well as testing the coating for compliance with international regulatory specifications.

**DESCRIPTION OF THE PRIOR ART**

Chlorhexidine is a bis-biguanide antiseptic and disinfectant that has bactericidal and bacteriostatic action against a wide range of gram-positive and gram-negative bacteria. Chlorhexidine has been used as a topical, antimicrobial tooth coating for the reduction of tooth decay in permanent teeth U.S. Patent No. 4,496, 322 describes a dental varnish containing an antimicrobial agent, specifically chlorhexidine diacetate/acetate, a benzoin gum, and an orally-acceptable solvent that, when applied to teeth, dries to a film, that provides sustained release of the antimicrobial agent. An improvement on this technology was described in U.S. Patent No. 4,883,534 which further provided a sealing composition, applied to the varnish, to extend the length of the antimicrobial protection provided by the varnish.

Tooth coating mixtures containing chlorhexidine have been sold commercially, for example, under the trademarks CHLORZOIN, under license from the University of Toronto, and PREVORA by CHX Technologies, Inc., Toronto, Canada. The PREVORA product is sold to skilled practitioners as a kit having two components: the first component (Stage 1) contains the active antimicrobial agent in a polymeric matrix of no therapeutic value, specifically Sumatra benzoin, and the second component (Stage 2) is a sealant comprising an aqueous dispersion of a polymer used to further increase the retention time of the therapeutic ingredients on the teeth.

Recently, the PREVORA tooth coating mixture has been approved for the reduction of dental caries in adults in Canada and Ireland. Thus, the PREVORA product must have the components and specifications required by appropriate international regulatory boards (*e.g.*, Health Canada, US Food and Drug Administration, and the European Agency for Evaluation of Medicinal Products). These specifications include, *inter alia,* potency of the active ingredient, pH, specific gravity, allowable content of "related substances" (impurities), and visual appearance. The following table summarizes the specifications of the finished product of PREVORA Stage 1 as approved by the Irish Medicines Board.

**Table A**

| Test | Specification |
|---|---|
| Description | A clear, slightly brownish solution with a characteristic medicinal odor, free of visible particulate matter |
| pH | 6.50-7.40 |
| Specific Gravity | 0.880- 0.910 |
| Contents by Volume | Average volume NLT 1 ml |
| Impurities | A. 4-chlorophenyl cyanamide/carbodiimide limit $\leq$ 0.1%<br>B. 4-chloroaniline limit $\leq$ 0.1%<br>C. 4-chlorophenyl isocyanate limit $\leq$ 0.1%<br>D. Total related substances limit $\leq$ 2.5% |
| Potency of chlorhexidine diacetate | 95.0 - 110.0 mg/ml |
| Total aerobic microbial count | NMT 100 cfu/ml |
| Total yeast and mold count | Determine and report |

Sumatra benzoin is the polymeric matrix of choice for the PREVORA Stage 1 and Stage 2 products. Many other varnishes, including cellulose acetate, ethyl cellulose, polycaprolactone, and Sandarac, have been investigated, but Sumatra benzoin has been found to be superior for retaining the composition on the teeth for the longest time. See, Masters Thesis of T. Balanyk, Development of sustained-release antimicrobial dental varnishes effective against Streptococcus mutans in vitro, University of Toronto, Faculty of Dentistry (1986). Sumatra benzoin is a natural resin that, of course, varies from batch to batch. The CHLORZOIN topical coating, for example, had been manufactured using a non-compendial grade Sumatra benzoin since as early as 1993. However, in order to comply with US and European regulatory requirements, the compendial-grade of Sumatra benzoin as specified in the current edition of the *British pharmacopoeia* and/or the *European Pharmacopoeia* must be used. The compendial grade of Sumatra benzoin is herein designated "Sumatra benzoin BP" to distinguish it from the non-compendial grade Sumatra benzoin used in the past. It is to be understood, however, that the term Sumatra benzoin BP also refers to a grade of Sumatra benzoin that also complies with the *European Pharmacopoeia.*

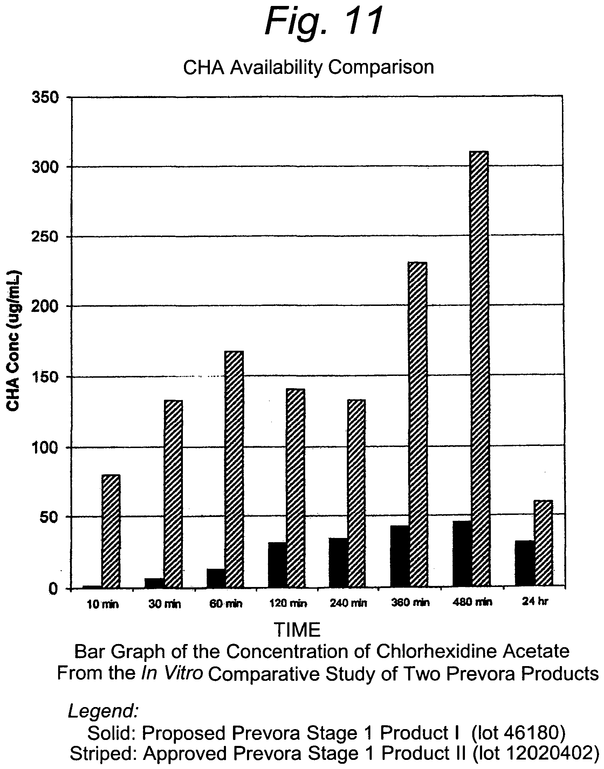

Surprisingly, the compendial grade, Sumatra benzoin BP, contains a large amount of particulates, in the nature of siftings from a can of mixed nuts, whereas the non-compendial house-grade Sumatra benzoin previously employed is a clear, thick viscous fluid, in the nature of honey. Interestingly, chromatographs of the two grades of Sumatra benzoin are completely different. Moreover, the release characteristics of chlorhexidine from a non-compendial Sumatra benzoin matrix versus a Sumatra benzoin BP matrix are different (see, Fig. 11 herein).

Use of Sumatra benzoin BP led to unanticipated difficulties in formulating the mixture of chlorhexidine in Sumatra benzoin BP and in ascertaining whether the finished product complies with regulatory requirements. There is, therefore, a need for an improved process of manufacturing a topical, antibacterial coating containing chlorhexidine in Sumatra benzoin BP.

In addition to the foregoing, there is a need for improved test procedures for establishing compliance with regulatory requirements, such as methods to evaluate the potency of chlorhexidine in the final topical solution, as well as to assess the content of "related substances" of chlorhexidine. Related substances are known degradation products. Two impurities, or degradation products, to chlorhexidine have been reported in the literature: 4-chlorophenyl isocyanate and 4-chlorophenyl carbodiimide (or 4-chlorophenylcynanamide). However, the *European Pharmacopoeia* lists additional impurities, designated EP Chlorhexidine Acetate Related Compounds A and C. Therefore, a new test had to be developed to detect these impurities in a finished product of the topical solution.

In addition to the foregoing, the *European Pharmacopeia* proposes a test method for determining the concentration of chlorhexidine diacetate in solution using high performance liquid chromatography (HPLC). However, the use of Sumatra benzoin BP as the matrix results in excessive noise (chatter) in HPLC chromatography, such that the peaks of chlorhexidine, and its related substances, are obscured. Therefore, there is a need for an improved methods of testing chlorhexidine in Sumatra benzoin BP that complies with the *European Phamacopoeia* test methods.

## Summary of the Invention

The foregoing and other objects are achieved by this invention which provides, in one embodiment, a novel method of manufacturing a topical, antibacterial coating containing chlorhexidine, as the active anti-bacterial agent, in a matrix of the natural substance, Sumatra benzoin BP, so that existing specifications for the drug product would not be changed.

As indicated above, Sumatra benzoin BP contains particulates not found in non-compendial Sumatra benzoin. The particulate limit for Sumatra benzoin BP, according to the existing standard, is 1.2 $\mu$m. Thus, additional filtration steps were required to reach the level of no visible particulate matter. Compared to prior methods using non-compendial Sumatra benzoin, up to five filtration steps are required. The additional filtration steps add time to the overall process and cause evaporative losses of solvent (ethanol), as well as losses of active ingredient that are retained on the filter media. This can compromise potency of the finished drug product, and therefore, a method of manufacturing, or compounding the finished drug product, had to be developed to compensate for this complication.

In accordance with this aspect of the invention, the compounding process for formulating a batch of finished drug product comprises the steps of:

forming a stock solution of Sumatra benzoin BP in a solvent, which in a particularly preferred embodiment is ethanol, by mixing the Sumatra benzoin BP in ethanol for a period of time just sufficient to dissolve the Sumatra benzoin BP;
filtering the stock solution to form a substantially particulate free stock solution;
mixing chlorhexidine diacetate in the filtered stock solution for a period of time just sufficient to dissolve the chlorhexidine; and
adding a quantity of ethanol sufficient to form a finished drug product of chlorhexidine diacetate in ethanolic Sumatra benzoin BP having the requisite potency of the active ingredient, pH, specific gravity, related substances content, and visual appearance to comply with current regulatory requirements, illustratively as set forth in Table A. Of course, the product should comply with existing standards for microbial content.

The step of filtering comprises at least three to five steps. The multiple step filtration process enables the use of compendial grade Sumatra benzoin BP, which as stated above, is necessary for compliance with current regulatory schemes. In a preferred specific embodiment, the stock solution is filtered through a series of filtration media, specifically a 2 mm strainer, a 300 $\mu$m filter, a 38 $\mu$m filter, and a 1.2 $\mu$m filter to remove insoluble material. The particulate limit for Sumatra benzoin BP, according to the existing standard, is 1.2 $\mu$m. In some embodiments, compressed nitrogen gas may be used to facilitate filtration through the final 1.2 μm filter. The result is a filtered, stock solution of chlorhexidine diacetate in Sumatra benzoin BP that is substantially free of particulates, and in compliance with the existing regulatory standard.

Compounding to form a finished product of chlorhexidine diacetate in ethanolic Sumatra benzoin BP is facilitated because the mixing times are based on peak solubility of the Sumatra benzoin BP in the solvent and of the chlorhexidine in the stock solution. As a practical mater, this makes up for the additional time required to accomplish the additional filtration steps in order to achieve a clear solution that is free of all particulates exceeding specifications.

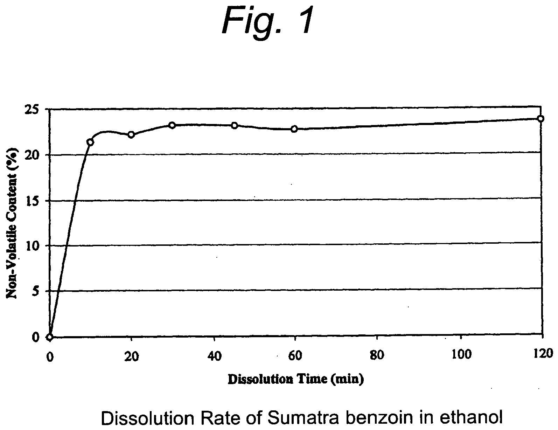

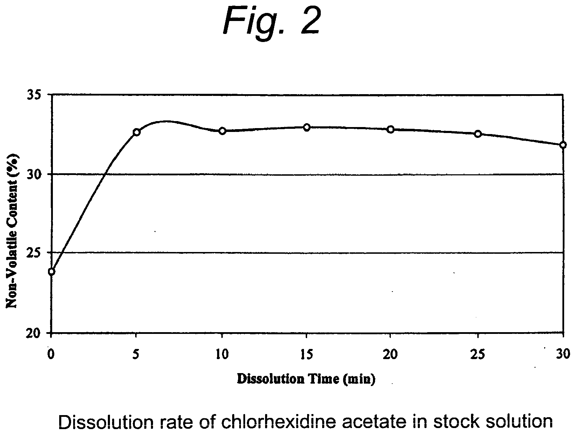

The mixing times are determined by dissolution studies of the type described in more detail in Example 1hereinbelow (see, Figs. 1 and 2). At a stirring speed of at least 500 rpm, for example, Sumatra benzoin BP is completely dissolved by 30 minutes. Similar studies are conducted to determined the dissolution time of chlorhexidine diacetate in a stock solution of alcoholic Sumatra benzoin BP show that the chlorhexidine diacetate is completely dissolved within 30 minutes. In a specific illustrative embodiment, Sumatra benzoin BP is mixed with ethanol for 30 minutes, with stirring at 1750 rpm to form a stock solution. Chlorhexidine diacetate was mixed into the stock solution and stirred, at 900 rpm, for 10 minutes. These parameters were ascertained to be adequate to completely dissolve the Sumatra benzoin BP and chlorhexidine diacetate, in the respective solutions.

The amount of Sumatra benzoin BP that is lost as insoluble matter during the multiple filtration steps is determined by weighing the non-volatile material collected on the filter media. In addition, the amount of solvent, or volatile component(s), lost during the filtration and other manufacturing steps is ascertained so that solvent can be added q.s. to bring the volume/weight to the desired amount.

In a product by process embodiment of the invention, a topical, antibacterial coating containing chlorhexidine in an ethanolic solution of Sumatra benzoin BP is made by the process described above. The resulting product, while retaining the same key regulatory specifications with respect to potency of the active ingredient (*i.e.,* the concentration of chlorhexidine diacetate in the finished drug product), pH, specific gravity, allowable content of "related substances" (impurities), and visual appearance, is distinct from a product incorporating non-compendial grade Sumatra benzoin, and manufactured according to pre-existing procedures.

After compounding, the finished drug product is tested for compliance with pertinent regulatory standards which, presently, are as set forth in Table A. In a further embodiment of the invention, methods of testing the bulk finished drug product solution for compliance with international regulatory specifications were developed based on existing compendial HPLC methods for chlorhexidine diacetate in solution relating to, *inter alia,* potency and "related substances," or the impurities which are the expected degradation products of chlorhexidine. However, compendial test methods, useful for assaying a particular substance, such as chlorhexidine, are not necessarily useful for assaying a compounded drug product containing the drug substance and other medicinal, or non-medicinal, ingredients that could interfere with the assay results.

For example, there are at least three known degradation products of chlorhexidine. These are 4-chloroaniline, 4-chlorophenyl isocyanate, and 4-chlorophenyl carbodiimide (also known as 4-chlorophenyl cyanamide). When testing a chlorhexidine solution that also contains Sumatra benzoin BP, however, existing HPLC methods could not identify two of the known degradation products: 4-chlorophenyl isocyanate, and 4-chlorophenyl carbodiimide due to interfering peaks, or chatter, in the chromatography. Moreover, the formulated product containing Sumatra benzoin BP includes additional impurities, designated EP chlorhexidine acetate related Compounds A and C. Compound A is 1-[4-chlorophenyl)-5-(6-(3-cyanoguanidino)hexy]biguanide and Compound C is p-chlorophenyl urea.

In a method of testing for potency and "related substances" embodiment, the final solution of chlorhexidine diacetate in ethanolic Sumatra benzoin BP is stripped of Sumatra benzoin prior to testing. An acidic aqueous medium is used as a selective solvent to precipitate all of the Sumatra benzoin resin from the compound formulation while leaving all of the chlorhexidine diacetate in solution. In a specific illustrative embodiment, the acidic aqueous medium comprises 5% phosphoric acid in water. There is 100% recovery of chlorhexidine diacetate.

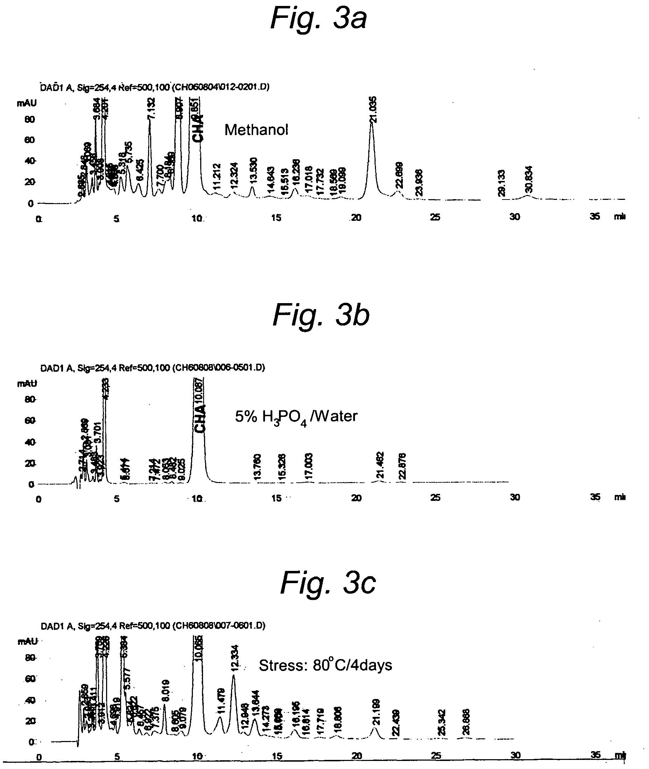

Prior art techniques utilizing an organic solvent, such as methanol, did not precipitate out the Sumatra benzoin resin. In this regard, Figs. 3A-3C show chromatograms of solutions of chlorhexidine diacetate in ethanolic Sumatra benzoin BP that have been diluted 100 x with methanol (Fig. 3A) and 100 x with 5% phosphoric acid in water (Fig. 3B).

Referring to Fig. 3A, all of the peaks, except the peak labeled "CHA" (for chlorhexidine diacetate) are from the Sumatra benzoin BP. In Fig. 3B, the majority of the extraneous resin peaks are gone except in the early part (up to 4.2 minutes) where there is no chlorhexidine diacetate. The chlorhexidine diacetate peak, labeled "CHA" is very clearly shown in Fig. 3B.

Fig. 3C is a chromatogram of a "stressed" product, that is where the final solution of chlorhexidine diacetate in ethanolic Sumatra benzoin BP has been held at a temperature of 80 ° C for four days and then prepared for testing by diluting in the same acidic aqueous medium (100 x with 5% phosphoric acid in water) used to generate Fig. 3B. Stressing accelerates the production of degradation products, and may include subj ecting the sample to heat, change of pH, oxidation with peroxide, and exposure to UV. As expected, Fig. 3C shows that degradation products have been generated, and the potency of chlorhexidine diacetate is only 50% due to stressing.

In a further embodiment of the invention, a method of manufacturing a topical, antibacterial coating containing chlorhexidine in Sumatra benzoin BP comprises the additional steps(s) of testing the finished drug product for compliance with international regulatory standards, including one or more of the following test procedures:

A) testing to confirm (or assay) concentration (mg/ml) of active ingredient, chlorhexidine acetate, in the finished drug product by an HPLC method as set forth in Example 2. In this HPLC method embodiment, the finished drug product is stripped of Sumatra benzoin BP prior to testing by using a selective solvent to precipitate substantially all of the Sumatra benzoin BP leaving only active drug chlorhexidine diacetate in solution. In a specific preferred embodiment, the selective solvent is a dilute acidic aqueous medium, and most preferably is 5% phosphoric acid.

B) testing to ascertain presence of known degradation products (or "related substances") of the active ingredient, which in the case of chlorhexidine, is 4-chloroaniline, 4-chlorophenyl carbodiimide and 4-chlorophenyl isocyanate. In one embodiment, a colorimetric procedure, as described in Example 5, is used to ascertain whether the amount of 4-chloroaniline in the finished drug product exceeds the permissible limit of 50 ppm. Specifically, 4-chloroaniline is determined by diazotizing nitrite in acid solution and coupling with napthylethylenediamine dihydrochloride to form a red-blue (purple) dye that is visually compared to the color produced by a standard solution containing an amount of 4-chloroaniline at the maximum permissible limit.

In another embodiment for ascertain the presence and amount of the related substances, 4-chlorophenyl carbodiimide and 4-chlorophenyl isocyanate comprises an HPLC method, in accordance with Example 4. In this specific preferred embodiment, 4-chlorophenylisocyanate is converted to N-4-chlorophenyl ethylcarbamate in an ethanol solution so that the generated spectral peaks avoids interference from the Sumatra benzoin BP.

C) testing to ascertain for total "related substances" content, that is, to ascertain the total amount of degradation products, specifically including chlorhexidine acetate Compounds A and C according to the HPLC method as set forth in Example 3. This preferred embodiment includes stripping the finished drug product of Sumatra benzoin BP prior to testing by using the selective solvent to precipitate substantially all of the Sumatra benzoin BP from solution.

Of course, in addition to the foregoing, the finished drug product is tested for compliance with other regulatory specifications, such as pH and specific gravity.

**Brief Description of the Drawing**

Comprehension of the invention is facilitated by reading the following detailed description, in conjunction with the annexed drawing, in which:

Fig. 1 is a graphic representation of the dissolution rate of Sumatra benzoin in ethanol shown as the percent of non-volatile content of Sumatra benzoin dissolved in ethanol plotted as a function of dissolution time in minutes;

Fig. 2 is a a graphic representation of the dissolution rate of chlorhexidine diacetate in a stock solution of Sumatra benzoin in ethanol shown as the percent of non-volatile content dissolved in stock solution plotted as a function of dissolution time in minutes;

Fig. 3A-3B are chromatograms of chlorhexidine diacetate in ethanolic Sumatra benzoin BP that have been diluted with an organic solvent, methanol (Fig. 3A); an acidic aqueous solvent, 5% phosphoric acid in water (Fig. 3B); and an acidic aqueous solvent, 5% phosphoric acid in water after being "stressed" at a temperature of 80 ° C for four days (Fig. 3C);

Fig. 4 is an HPLC chromatogram obtained in accordance with the parameters set forth in Example 2, of a blank comprising a 5% phosphoric acid ($H_3PO_4$) (diluent);

Fig. 5 is an HPLC chromatogram in accordance with the parameters set forth in Example,2, of a working standard comprising chlorhexidine diacetate in diluent at a concentration of 0.5 mg/ml;

Fig. 6 is an HPLC chromatogram in accordance with the parameters set forth in Example,2, of a sample of a finished drug product made in accordance with Example 1;

Fig. 7 is an HPLC chromatograms, obtained in accordance with the parameters of Example 3 of a blank comprising a 5% phosphoric acid ($H_3PO_4$) (diluent);

Fig. 8 is an HPLC chromatogram in accordance with the parameters set forth in Example 3 of a standard comprising a 2.5% solution of chlorhexidine diacetate in diluent;

Fig. 9 is an HPLC chromatogram in accordance with the parameters set forth in Example 3 of a resolution solution of 1.5 mg/ml EP chlorhexidine CRS in diluent;

Fig. 10 is an HPLC chromatogram in accordance with the parameters set forth in Example 3 of a sample (PREVORA Stage 1 Placebo Solution); and

Fig. 11 is a bar graph of chlorhexidine (CHA) availability as a function of concentration ($\mu$g/ml) *in vitro* plotted against time (minutes) for chlorhexidine diacetate in ethanolic Sumatra benzoin version 1 (solid) and version 2 (striped).

**Detailed Description**

Compounding

In order to use the compendial-grade Sumatra benzoin BP, a stock solution of Sumatra benzoin BP in ethanol was first made by dissolving Sumatra benzoin BP in ethanol and filtering same in order to reduce the particulates to the requisite standard. Then the active agent, chlorhexidine diacetate was dissolved in the stock solution. Solvent, specifically ethanol, was added q.s. to bring the solution to the proper concentration. The result is a final solution of the Stage 1 component of the PREVORA kit containing the active ingredient chlorhexidine diacetate.

Table 1 summarizes the composition of the two components (identified as Stages) of the PREVORA kit for topical preventive treatment for adult tooth decay. Stage 1 is a topical antibacterial coating containing the active ingredient, chlorhexidine, Stage 2 is a sealing varnish used to prolong retention of the Stage 1 product on the teeth. The regulatory standard for the component ingredients are also listed in Table 1.

Table 1

| Ingredient | Regulatory Standard | Concentration |
|---|---|---|
| Topical Dental Coating (Stage 1) | | |
| Chlorhexidine diacetate | EP | 10% (w/v) (100 mg/ml) |
| Sumatra Benzoin | BP | 20% w/v (200 mg/ml) |
| Ethyl Alcohol | USP/EP | q.s. to 1 ml |
| Sealant (Stage 2) | | |
| Eudragit RS 30D (aqueous acrylic dispersion, available from Rohm Pharma Polymers of Rohm GmbH, Darmstadt, Germany | NF | 94% w/w |
| Triethyl Citrate (plasticizer) | USP | 6% w/w |
| EP - European Pharmacopoeia<br>BP - British Pharmacopoeia<br>USP - US Pharmacopendium<br>NF - Non-Formutary | | |

Example 1:

In a specific preferred embodiment of a method of making aspect of the present invention, a 12 kg batch of chlorhexidine diacetate in a stock solution of ethanolic Sumatra benzoin BP is prepared as follows:

1) A stock solution of Sumatra benzoin BP in ethanol is prepared by adding 2.682 kg of benzoin siftings to 7.911 kg of 100% ethanol. The mixture is stirred at 500 rpm for 30 minutes to form a stock solution that contains particulate matter due to the compendial grade of Sumatra benzoin BP.
2) the solution is filtered through a series of filtration media, specifically a 2 mm strainer, a 300 $\mu$m filter, a 38 $\mu$m filter, and a 1.2 $\mu$m filter to remove insoluble material. Compressed nitrogen gas may be used to facilitate filtration through the final 1.2 $\mu$m filter. The result is a filtered, stock solution containing no particulates of greater that 1.2$\mu$m diameter.
3) chlorhexidine diacetate (1.408 kg) is added to the filtered, stock solution and stirred for 10 minutes at 500 rpm.
4) Ethanol is added to the solution resulting from step (3) so that the final solution, or finished product, weighs 12 kg. The final solution is mixed for an additional 10 minutes.

The data presented hereinbelow demonstrated how the optimum mixing times were calculated for both the stock solution and the final solution from the dry weights of the non-volatile content in 5 ml samples.

Dissolution Studies

The length of time required to dissolve Sumatra benzoin BP in alcohol, at room temperature, was ascertained by the following method.

A solution of Sumatra benzoin BP was prepared by weighing 1950.2 g of 100% ethanol into a 4 liter beaker and adding 661.14 g of Sumatra benzoin BP. A stirrer was set to 500 rpm and started. After 10 minutes, the stirring was stopped, the solution was allowed to settle the suspended solids for one minute, and a sample was drawn into a 5 ml syringe through a 10 micron stainless steel screen to filter out any solids remaining in suspension. This procedure was repeated and samples were withdrawn at 20, 30, 45, 60, and 120 minutes.

Non-volatile content of the withdrawn samples was determined by weighing the solution before and after evaporating the ethanol solvent for 1 hour at 110°C. Fig. 1 shows the non-volatile content, plotted as a function of time. As shown in Fig. 1, Sumatra benzoin BP is almost completely dissolved within 10 minutes, while full dissolution is achieved by about 30 minutes at a mixing speed of at least 500 rpm.

A solution of chlorhexidine diacetate was prepared by adding 313.1 g of chlorhexidine diacetate to 2,309.5 g of the filtered Sumatra benzoin BP stock solution. Using a technique similar to that described above in connection with the dissolution rate of Sumatra benzoin BP in ethanol, a stirrer was set to 500 rpm and started. Samples were taken in 5 minute intervals over a 30 minute period. Non-volatile content of the withdrawn samples was determined by weighing the solution before and after evaporating the solvent. Fig. 2 shows the non-volatile content, plotted as a function of time. As shown in Fig. 2, the chlorhexidine diacetate was completely dissolved within about 5 minutes. The final non-volatile content was measured at 30.2% as compared to a theoretical value of 32.9%. The difference is due to additional ethanol that was added after dissolution of the chlorhexidine to make up for the loss of the insoluble fraction of the Sumatra benzoin BP .

Filtration Studies

In order to evaluate the effectiveness of a filtration process to remove particulates in the Sumatra benzoin BP solution, a stock solution of Sumatra benzoin BP was filtered using a multi-stage process through a series of filtration media. The filtration medium was weighed before filtration. The stock solution was stirred to suspend any undissolved matter and poured through the filter. When the solution finished draining, the filtration medium and retained undissolved particulates, were dried and re-weighed. In some instances, fine, insoluble material plugged the 1.2 $\mu$m polycarbonate filter, so compressed nitrogen was used to force the ethanol through the filter. The final filtrate was clear and free of sediment.

The results, in the form of the weight and percentage of total undissolved matter, selected at each stage, are tabulated in Table 2 below.

Table 2

| Undissolved Matter Collected at Each Filtration Stage | | |
|---|---|---|
| Filter Pore Size | Weight of Undissolved Matter | % of Total Undissolved Matter |
| 4 mm (~6 mesh) | 28.35 | 54.9 |
| 2 mm (~12 mesh) | 11.49 | 22.3 |
| 200 $\mu$m | 6.46 | 12.5 |
| 146 $\mu$m | 1.18 | 2.3 |
| 38 $\mu$m | 1.51 | 2.9 |
| 1.2 $\mu$m | 2.62 | 5.1 |

As shown on Table 2, about 77% of the total insoluble material was collected on the 2 mm mesh, while the 300 $\mu$m filter collected another 12.5%. The 146 $\mu$m screen only collected about 2.3% of the insoluble particulates, but these insoluble particulates were filtered out by the 38 $\mu$m filter. In view of the foregoing, a multi-stage filtration process was employed in a practical embodiment of the invention consisting of four stages: 2 mm, 300 $\mu$m, 38 $\mu$m, and 1.2 $\mu$m to remove all insoluble material.

After filtration, the final weight, non-volatile content, and pH of the stock solution of Sumatra benzoin BP in ethanol (Example 1) were measured. The composition of the filtered stock solution is 1,759.1 g ethanol and 550.4 g Sumatra benzoin BP, in this example, for a total of 2,309.5 g.

Approximately 300 g of stock solution was lost in the manufacturing process by evaporation of ethanol during mixing and filtration, removal of solution for measurement of non-volatile content and loss of solution during filtration due to transfer of solutions and spillage. About 7.7% of the Sumatra benzoin BP (0.207 kg) was lost as insoluble material during filtration. The properties of the final stock solution are as follows: non-volatile content of 23.83%, specific gravity (ASTM D) of 0.8733, and pH of 5.26.

The foregoing specific illustrative method of making a topical dental solution comprising chlorhexidine in an ethanolic solution of Sumatra benzoin BP (*e.g.,* PREVORA Stage 1) is summarized in Table 3. Table 3 also shows the process difference between a specific illustrative method used, also successfully, for manufacturing a topical dental solution using Sumatra benzoin (non-compendial; e.g., CHLORZOIN).

**Table 3**

| Differences in Methods of Manufacture | | |
|---|---|---|
| Ingredient/Method Step | Process Using Sumatra benzoin (non-compendial) | Process Using Sumatra benzoin BP |
| BULK MANUFACTURING | | |
| Grade of Sumatra benzoin | Non-Compendial/Mod. USP | BP |
| Grade of chlorhexidine | BP | EP |
| Stock Solution Preparation | 3 hrs in a mixer at 60 psi | 30 minutes, stirred at rt and atm. pressure at 1750 rpm |
| Filtration of Stock Solution | --- | 4 progressive filtration steps to final 1.2$\mu$m |
| Addition of Drug Substance | added to unfiltered stock solution | added to filtered stock solution |
| Mixing of Drug Substance with Stock Solution | 10 minutes in a mixer at 60 psi | 10 minutes, stirred at rt and atm. pressure at 900 rpm |
| qs with alcohol | 35 minutes in mixer | 5 minutes, stirred at rt and atm. pressure at 900 rpm |
| Filtration of Final Drug Solution | 1.2$\mu$m filter with N pressure at 5-10 psi | --- |

Testing Procedures

An analytical procedure was developed for the determination of the concentration of chlorhexidine in the PREVORA Stage 1 product as an assay for potency of the active ingredient. This method is a modification of a procedure used for the determination of "related substances" described in the current EP Monograph for chlorhexidine diacetate (European Pharmacopoeia, 5th Ed., chlorhexidine Diacetate, monograph 0657). In lieu of the organic solvents typically used as a diluent, and more particularly methanol, a weak aqueous acid diluent was used to precipitate out all of the Sumatra benzoin resin from the compound formulation while leaving 100% of the chlorhexidine diacetate in solution.

In a particularly preferred embodiment, the weak aqueous acid diluent is 5% phosphoric acid ($H_3PO_4$) (v/v) made by diluting 100 ml 85% phosphoric acid to 2.0 liters with water. Studies were conducted using other acids, such as acetic, formic, trifluoroacetic, and hydrochloric, concentrations up to 10%. Optimum results were achieved, however, with phosphoric acid at 5%.

A specific illustrative embodiment of the HPLC process for determining the concentration of chlorhexidine diacetate in the finished product is set forth below in Example 2:

Example 2:

1) HPLC equipment and Parameters:

| | | |
|---|---|---|
| HPLC System | : | Waters Alliance, Agilent 1100 or equivalent |
| Column | : | Alltech Alltima C-=18; 4.6 mm x 250 mm, 5$\mu$m |
| Flow Rate | : | 1.0 ml/min |
| Injection Vol. | : | 10 $\mu$l |
| Detection Wavelength | : | 254 nm |

(continued)

| Mobile Phase A | : | 12% Acetic acid and 2% sodium octanesulphonate in water:methanol, 27:73 (v/v). A stock solution of sodium octanesulphonate-acetic acid is prepared by dissolving 2.0 g sodium octanesulphonate in 270 ml of water and mixing with 730 ml of methanol followed by 120 ml of glacial acetic acid. The mixture is filtered through a 0.45 $\mu$m nylon filter and degassed prior to use by sonication. |
| Run Time | : | 30 minutes |
| Diluent | : | 5% phosphoric acid ($H_3PO_4$) (v/v) made by diluting 100 ml 85% phosphoric acid to 2.0 liters with water |
| Col. Temperature | : | Ambient |
| Sample Temperature | : | Ambient |

2) Sample and Standard Preparation

Sample Solution Preparation

A 1.0 ml sample of the finished drug product is diluted with the diluent in a 200 ml volumetric flask, sonicated for 30 minutes, and filtered through a 0.45 $\mu$m Teflon syringe filter, 13 mm , or equivalent. In the alternative, an aliquot of the sample may be centrifuged at high speed (*e.g.*, 10,000 rpm) for 30 minutes.
Two working standard solutions (0.5 mg/ml) were prepared by accurately weighing 50 mg of chlorhexidine diacetate reference standard into 100 ml volumetric flask, adding diluent, and mixing well.

3) System Suitability and Sample Analysis

Sequence:

inject blank (diluent) at least once
inject working standard 1 five times
inject working standard 2 one time
inject sample solution(s) once apiece

From the injections of working standard solution, % RSD (Relative Standard Deviation) was calculated for the area of the chlorhexidine peak. From the chlorhexidine peak, the USP Plate Count (tangent) and USP Tailing Factor were calculated.
The chlorhexidine diacetate (CHA) concentration in the sample solution(s) are calculated using the following equation:

$$CHA\ (mg/ml) = = (A_{smpl}/A_{Std}) \times (W_{std}/1\ ml) \times DF \times 100$$

where:

$A_{smpl}$ = peak area of chlorhexidine peak in the sample
$A_{Std}$ = mean of chlorhexidine peak area in the standard (system suitability)
$W_{std}$ = weight of standard in mg (corrected for potency)
DF = dilution factor = sample dilution/standard dilution

To meet specifications, the concentration of CHA in a finished drug product, such as the drug product described in Example 1 must be between 100.0 to 110.0 mg/ml.

Acceptance criteria:

> Working standard solution (5 injections)
> chlorhexidine peak area %RSD (5-injections) NMT 2.0%
> USP Plate Count (tangent): NLT 2000
> USP Tailing Factor: NMT 2
> Working standard 2 check:
> % recovery of standard 2 = 98-102%

4) Results

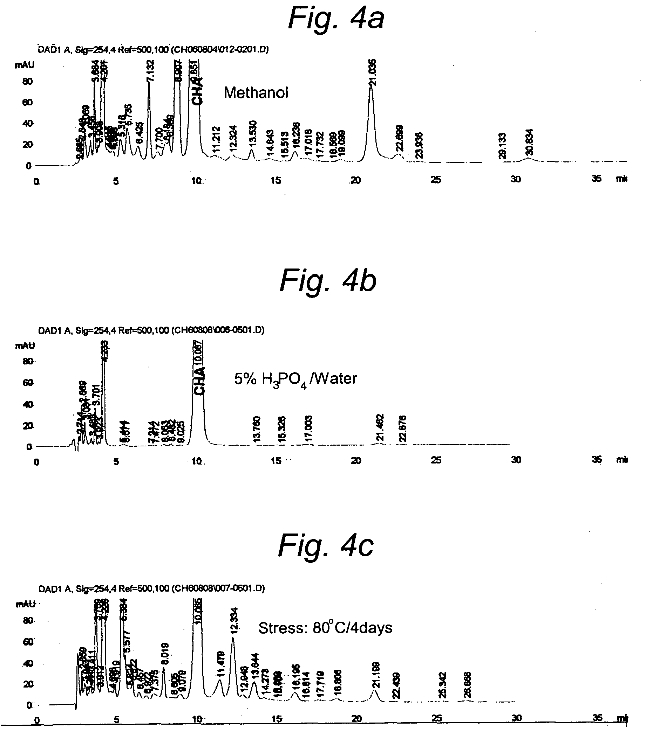

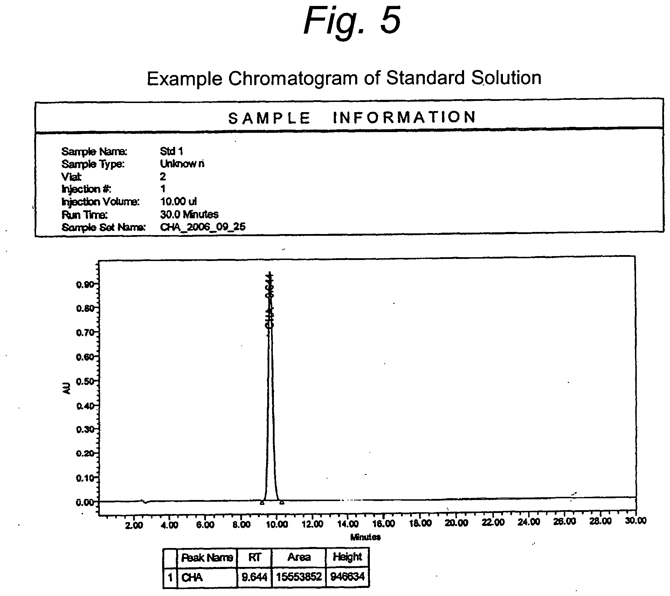

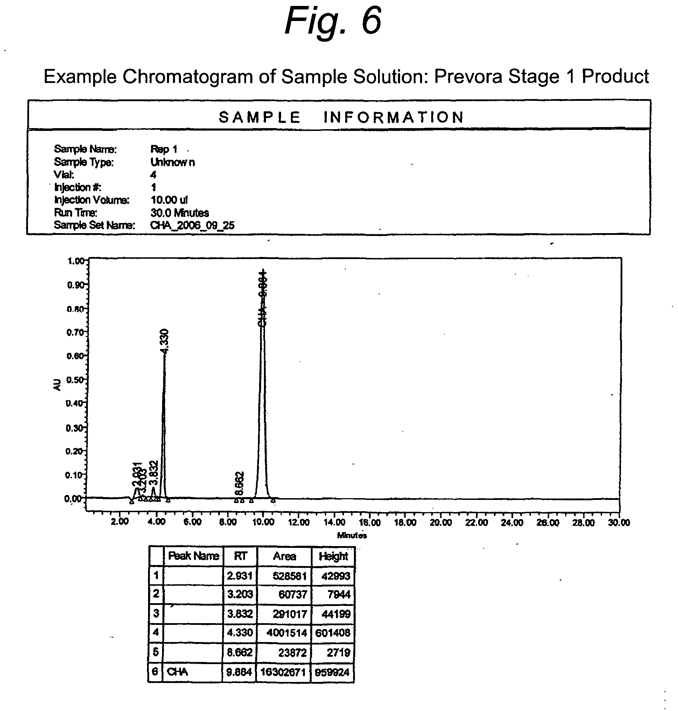

The results are shown in Figs. 4,5, and 6 which are, respectively, exemplary HPLC chromatograms obtained in accordance with the parameters set forth in Example 2 of blank (diluent); working standard 1, and a sample of the finished product. Figs. 4 to 6 include retention time (RT), Area, and Height data for each named peak.

The HPLC process for determining the concentration of chlorhexidine diacetate of Example 2 was subjected to validation studies, including specificity (non-interference from matrix components, mobile phases, diluent and potential degradation products), precision (*e.g.*,, repeatability), accuracy, range (linearity), robustness (ability to perform as intended with minor changes to mobile phase composition, column temperature and, and methanol:water ratio in mobile phase); filter compatibility (filtered versus unfiltered sample solutions), and standard and solution stability over time at ambient temperature). All of the studies resulted in satisfactory performance.

In further testing embodiment of the invention, there is additionally provided an HPLC method of detecting the presence of known degradation products (or "related substances") of the active ingredient in the finished drug product. In the case of chlorhexidine, the known related substances are 4-chloroaniline, 4-chlorophenyl carbodiimide and 4-chlorophenyl isocyanate. In finished drug product containing Sumatra benzoin BP there are additional known impurities, designated EP chlorhexidine acetate related Compounds A and C.

A specific illustrative embodiment of an HPLC process for determining the presence and amount of related substances of chlorhexidine diacetate in the finished product, and specifically Compounds A and C, is set forth below in Example 3:

Example 3:

1) HPLC Equipment and Parameters:

| | | |
|---|---|---|
| HPLC System | : | Waters Alliance, Agilent 1100 or equivalent |
| Column | : | Alltima C-18; 4.6 mm x 250 mm, 5$\mu$m |
| Flow Rate | : | 1.0 ml/min |
| Injection Vol. | : | 20 $\mu$l |
| Detection Wavelength | : | 254 nm |
| Mobile Phase A | : | 12% Acetic acid and 2% sodium octanesulphonate in water:methanol, 50/50/ (v/v). A stock solution of sodium octanesulphonate-acetic acid is prepared by mixing 4.0 g sodium octanesulphonate into 1000 ml of water, filtering (through 25 mm extraction disc), and adding 240 ml glacial acetic acid. Mobile Phase A is prepared by mixing 620 ml of the stock solution with 5000 ml of methanol. The mixture is degassed prior to us by filtration through a 0.45 $\mu$m nylon filter or by sonication. |
| Mobile Phase B | : | 12% Acetic acid and 2% sodium octanesulphonate in water:methanol, 20/80/ (v/v). Mobile Phase B is prepared by mixing 620 ml of stock solution with 40 ml of glacial acetic acid and 800 ml of methanol. The mixture is degassed prior to use by filtration through a 0.45 $\mu$m nylon filter or by sonication. |
| Gradient Program | : | See Table 4 hereinbelow |
| Run Time | : | 40 minutes |

(continued)

1) HPLC Equipment and Parameters:

| | | |
|---|---|---|
| Diluent | : | 5% phosphoric acid ($H_3PO_4$) (v/v) made by diluting 100 ml 85% phosphoric acid to 2.0 liters with water |
| Col. Temperature | : | Ambient |
| Sample Temperature | : | 4° C |

Table 4

| Gradient Program | | | |
|---|---|---|---|
| Time (min.) | Mobile Phase A (%) | Mobile Phase B (%) | Elution |
| 0 | 100 | 0 | equilibration |
| 0-10 | 100 → 66 | 0 → 34 | linear gradient |
| 10-15 | 66 → 0 | 34 → 100 | linear gradient |
| 15-29 | 0 | 100 | isocratic |
| 29-20 | 0 → 100 | 10 → 0 | linear gradient |
| 20-40 | 100 | 0 | re-equilibration |

2) Sample and Standard Preparation

A 1.0 ml sample of finished drug product is diluted to volume with the diluent in a 100 ml volumetric flask, sonicated for 30 minutes, and filtered through a 0.45 $\mu$m Teflon syringe filter, 13 mm, or equivalent. In the alternative, an aliquot of the sample may be centrifuged at high speed (*e.g.*, 10,000 rpm) for 30 minutes. A similar solution was prepared with a placebo. All prepared solutions should be stored under refrigerated conditions.

A resolution solution was prepared by dissolving 1.5 mg/ml EP chlorhexidine for performance test CRS in diluent.

A reference standard solution was prepared at 2.5% of the sample concentration. A stock solution of chlorhexidine diacetate reference standard at a concentration of 1 mg/ml in diluent was made by dissolving 100 mg of the chlorhexidine diacetate reference standard in 100 ml diluent. A standard (working) solution was prepared by diluting 5 ml of the stock solution of chlorhexidine diacetate reference standard with 200 ml diluent.

3) System Suitability and Sample Analysis

Sequence:

inject blank (diluent) at least once
inject resolution solution once
inject standard solution at 2.5% six times
inject sample solution(s) once apiece
inject placebo solution once for identification of placebo peaks
inject Standard Solution @2.5% (Check standard) once

The %RSD for the area of the chlorhexidine (CHA) peak was calculated from the six injections of the standard solution @ 2.5%. The % recovery of the recovery check injection was determined relative to mean of the six injections of standard solution.

4) Results

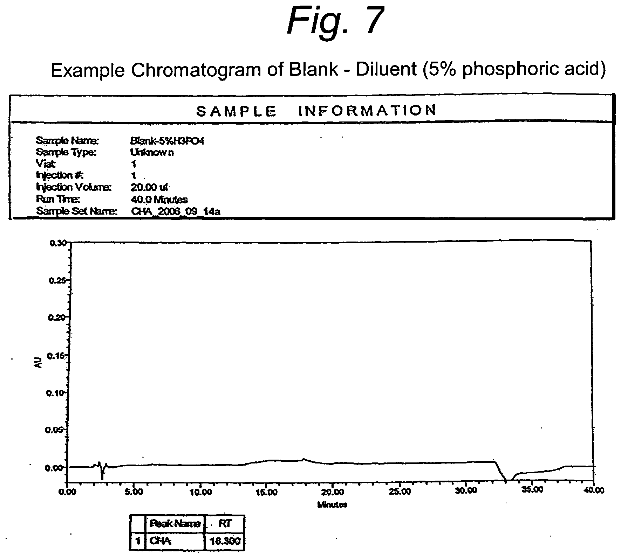

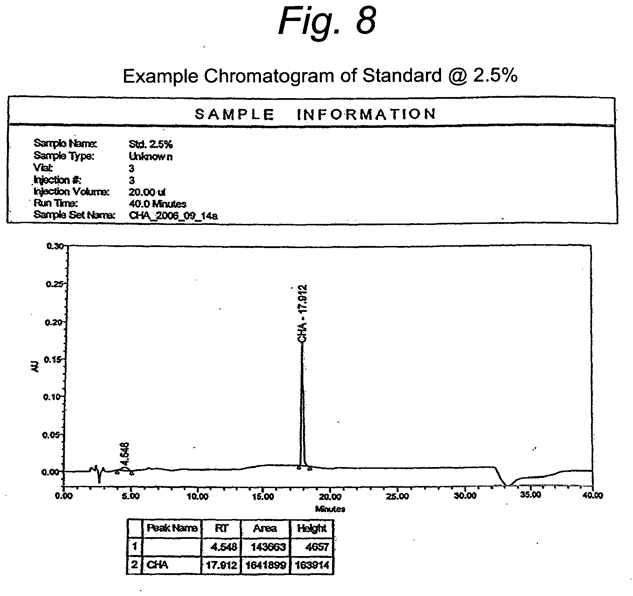

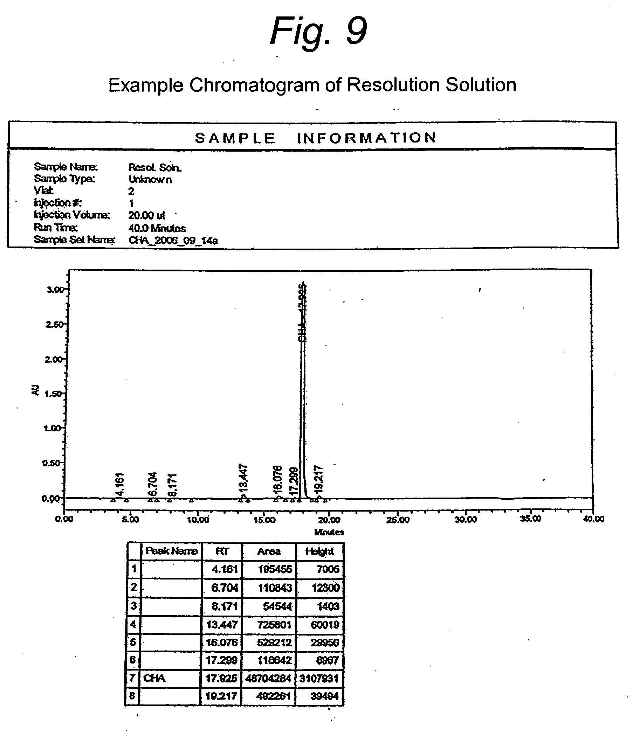

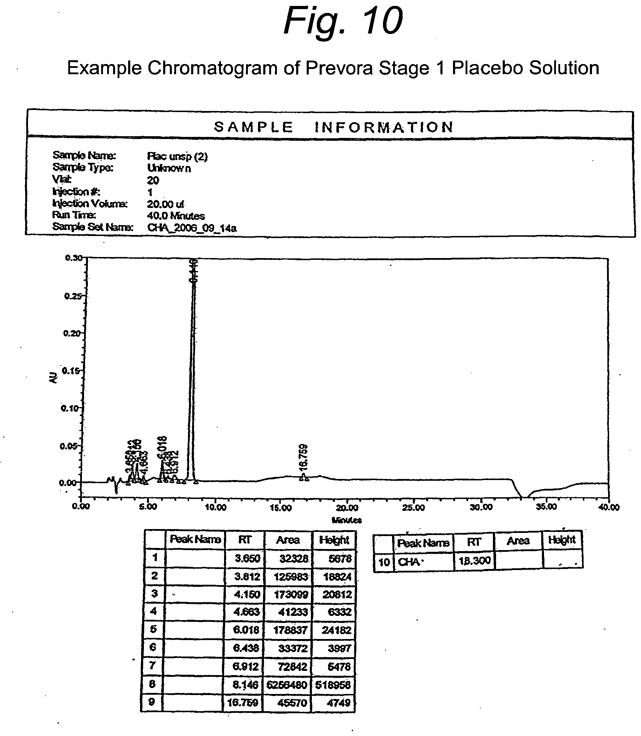

The results are shown in Figs. 7 to 10, which are, respectively, HPLC chromatograms, obtained in accordance with the parameters of Example 3, of blank (diluent); standard at 2.5%; resolution solution; and sample (Prevora Stage 1 Placebo Solution). Figs. 7 to 10 include retention time (RT), Area, and Height data for each named peak. The resolution solution chromatogram should look like the resolution solution chromatogram shown in Fig. 9.

Referring to Table 5, HPLC Data for peak identification is given for chlorhexidine diacetate and related Compound A and Compound C, as well as unknown(s). The peaks are identified according to their retention time (RT), relative retention time (RRT), and relative response factor (RRF). In order to comply with specifications for finished drug product, there should be no unknown impurity peaks larger than the reporting limit of 0.05% (calculated using the Standard @ 2.5%). Likewise, the amount of EP related substances, Compounds A and C, should not exceed 0.1%.

Peaks from the blank and placebo solutions are not included as impurity peaks. For example, impurity peaks larger than the reporting limits of 0.05% (calculated using standard at 2/5%) are used to calculate the amount of individual impurity using the following equation:

$$\% \text{ impurity (w/v)} = (A_{imp}/A_{Std}) \times (C_{std}/C_{smpl}) \times RRF \times 100$$

where:

$A_{imp}$ = peak area of impurity in the sample
$A_{Std}$ = mean of chlorhexidine peak area in the standard (system suitability)
$C_{std}$ = Concentration of Standard in mg/ml (corrected for potency)
$C_{smpl}$ = Nominal Concentration of working sample = 1.0 mg/ml
RRF = Response Factor for impurity (See Table 4)

Table 5

| HPLC Date for Peak Determination | | | |
|---|---|---|---|
| Peak Name | Typical RT (min.) | Relative RT | Relative Response Factor |
| chlorhexidine diacetate (CHA) | ~18.0 m in. | 1.0 | 1.00 |
| Unknown Peak(s) | Various | Various | 1.00 |
| EP chlorhexidine Related Substance Compound A | ~13.8 | 0.8 | 2.30 |
| EP chlorhexidine Related Substance Compound C | ~22.2 | 1.2 | 0.82 |
| RT = Retention Time. In the present case, determined from the average retention time of chlorhexidine working standard injections<br>Relative RT = RT of Impurity/RT of CHA<br>Relative Response Factor (RRF) | | | |

When testing for the presence of related substances 4-chlorophenyl isocyanate and 4-chlorophenyl carbodiimide in a solution that also contains Sumatra benzoin BP, however, existing HPLC methods could not identify these two of the known degradation products interfering peaks, or chatter, in the chromatography. Due to the high reactivity of isocyanate with alcohols, the quantitation of 4-chlorophenylisocyanate has been done by derivitization. The impurity, 4-chlorophenylisocyanate (CPI) readily converts to N-4-chlorophenyl ethylcarbamate in ethanol solution. The resulting carbamate is relatively stable and can be quantified by HPLC.

A specific illustrative embodiment of the test procedure for determining the presence of the related substances, 4-chlorophenylisocyanate and 4-chlorophenylcyanamide, of chlorhexidine acetate in the finished product is set forth below in Example 4:

Example 4:

1) HPLC Equipment and Parameters:
HPLC System        :        Waters Alliance, Agilent 1100 or equivalent

(continued)

1) HPLC Equipment and Parameters:

| | | |
|---|---|---|
| Column | : | Alltima C-18; 4.6 mm x 250 mm, 5$\mu$m |
| Flow Rate | : | 1.5 ml/min |
| Injection Vol. | : | 20 $\mu$l |
| Detection Wavelength | : | Detection Wavelength Program as follows |

| Time (min. | Wavelength (nm) |
|---|---|
| 0-11 | 260 |
| 11-25 | 240 |

| | | |
|---|---|---|
| Mobile Phase A | : | 0.1% formic acid in water is prepared by mixing 1.0 ml of formic acid with 1000 ml of water. The solution is degassed by filtration through an 0.24 $\mu$m nylon filter, or in the alternative, by sonication |
| Mobile Phase B | : | 0.1% formic acid in acetonitrile/methanol 90/10 (v/v) is prepared by mixing 1.0 ml formic acid with 900 ml of acetonitrile and 100 ml of methanol. The solution is degassed by filtration through an 0.24 $\mu$m nylon filter, or in the alternative, by sonication |
| Gradient Program | : | See Table 6 hereinbelow |
| Run Time | : | 25 minutes |
| Diluent 1 | : | Acetonitrile/Water (80:20) (v/v) is made by diluting 800 ml of acetonitrile with 200 ml of water |
| Diluent 2 | : | 5% phosphoric acid in water is made by diluting 100 ml of 85% phosphoric acid to 2.0 liters with water |
| Col. Temperature | : | Ambient |
| Sample Temperature | : | Ambient |

Table 6

| Gradient Program | | |
|---|---|---|
| Time (min.) | Mobile Phase A (%) | Mobile Phase B (%) |
| 0 | 75 | 25 |
| 5.0 | 75 | 25 |
| 16.0 | 50 | 50 |
| 21.0 | 25 | 75 |
| 22.0 | 25 | 75 |
| 22.1 | 75 | 25 |
| 25.0 | 75 | 25 |

2) Sample and Standard Preparation

Sample Solution for Analysis of 4-Chlorophenylcyanamide (Sample Solution 4A)

Allow the sample solution to come to ambient temperature for about 1 hour, if refrigerated. A 1.0 ml sample of finished drug product is placed into a 100 ml volumetric flask. It is important to allow adequate time for complete transfer of fluid into the flask. About 50 ml of Diluent 2, above, is mixed into the sample, and then brought to volume with more Diluent 2. The mixture is sonicated for 15 minutes and mixed well. The solution is heated in a water bath set at 40°C for 1 hour. The solution should then be permitted to come to room temperature and filtered through a 0.45 $\mu$m Teflon syringe filter, 13 mm, or equivalent. The sample solution should be stored at ambient temperature.

Sample Solution for Analysis of 4-Chlorophenylisocyanate (Sample Solution 4B)

Allow the sample solution to come to ambient temperature for about 1 hour, if refrigerated. A 1.0 ml sample of finished drug product is placed into a 100 ml volumetric flask. It is important to allow adequate time for complete transfer of fluid into the flask. Anhydrous ethanol (20 ml) is added to the flask, mixed, and then diluted to volume with water. The mixture is sonicated for 15 minutes and mixed well. It is then filtered through a 0.45 $\mu$m Teflon syringe filter, 13 mm, or equivalent. The sample solution should be stored at ambient temperature.

Reference standard solutions were prepared as follows:

CHA Standard Stock Solution (1 mg/ml in Diluent 1) is prepared by dissolving 100 mg of chlorhexidine acetate in 100 ml of Diluent 1. This solution should be stored under refrigeration.

CHA Standard Working Solution (0.005 mg/ml in Diluent 1) is prepared by diluting 1 ml of the CHA Standard Stock Solution with200 ml of Diluent 1. This solution may be stored at ambient temperature.

3) System Suitability and Sample Analysis

Sequence:

inject blank (Diluent 1) at least once
inject standard solution six times
inject Sample Solution 4A once
inject Sample Solution 5B once
inject Standard Solution (Check standard) once

The %RSD for the area of the chlorhexidine (CHA) peak is was calculated from the six injections of the standard solution. To be acceptable, the %RSD of CHA peak area should be NMT 5% and the % recovery from check standard should be 90-110%.

4) Results

Chromatograms were obtained, in accordance with the parameters of Example 4 of the standard solution, and Sample Solution 4A and Sample Solution 4B so that retention time (RT), Area, and Height data for each named peak could be obtained.

Referring to Table 7, the HPLC Data for peak identification is given for chlorhexidine acetate and related substances 4-chlorophenylcyanamide and 4-chlorophenylisocynate as N-4-chlorophenyl ethylcarbamate.

Table 7

| HPLC Data for Peak Determination | | | |
|---|---|---|---|
| Peak Name | Typical RT (min.) | Relative RT | Maximum UV absorbance (nm) |
| chlorhexidine acetate (CHA) | 9 min. | 1.0 | 259 |
| 4-chlorophenylcyanamide | 13.0 | 1.4 | 238 |
| 4-chlorophenylisocyanate | 18.0 | 2.0 | 242 |
| RT = Retention Time. In the present case, determined from the average retention time of chlorhexidine working standard injections<br>Relative RT = RT of Impurity/RT of CHA | | | |

The peaks are identified according to their retention time (RT), relative retention time, and maximum UV absorbance. In order to comply with specifications for finished drug product, the amount of EP related substances, 4-chlorophenylcyanamide and 4-chlorophenylisocynate, should not exceed 0.1%, individually.

For each sample, the amount of individual impurity can be calculated using the following equation:

$$\% \text{ impurity (w/v)} = (A_{imp}/A_{Std}) \times (W_{std}/1ml) \times 0.2 \times RF \times 100$$

where:

$A_{imp}$ = peak area of impurity in the sample
$A_{Std}$ = mean of chlorhexidine peak area in the standard (system suitability)
$W_{std}$ = Weight of chlorhexidine Standard in mg (corrected for potency)
0.2 = sample Dilution/Standard Dilution
RF = Response Factor for impurity

In addition to the foregoing HPLC test methods, a colorimetric method was developed to determine 4-chloroaniline content in the finished drug product. The permissible limit for the related substance 4-chloroaniline in the finished product is NMT 50 ppm (50 $\mu$g/ml).

In this embodiment, 4-chloroaniline is determined by diazotizing nitrite in acid solution and coupling with napthylethylenediamine dihydrochloride (NED) to form a red-blue (purple) dye. Any red-blue color developed in a sample solution is compared visually with a chloroaniline standard solution treated in a similar fashion. A specific illustrative embodiment of the test procedure for 4-chloroaniline is set forth in Example 5.

Example 5:

1) Sample Preparation

A 2 ml sample of finished drug product is placed into a 50 ml volumetric flask, diluted with 23 ml water, and mixed well to make the sample solution.

2) Standard Preparation

A 4-chloroaniline stock standard at 1000 $\mu$g/ml (0.10 g/1) is prepared by accurately weighing out 100 mg of 4-chloroaniline. The 4-chloroaniline is placed in a volumetric flask and diluted to volume with methanol and mixed well.

A 4-chloroaniline working standard at 10 $\mu$g/ml (0.01 g/l) is prepared by diluting 1 ml of the 4-chloroaniline stock standard to 100 ml with dilute hydrochloric acid (20% w/v).

A 4-chloroaniline comparison standard solution is made by pipetting 10 ml of 4-chloroaniline working standard into a 50 ml volumetric flask and adding 20 ml of dilute hydrochloric acid.

Reagent blanks for the standard solution and the sample solution are dilute hydrochloric acid (20% w/v; 30 ml).

All of the sample and standards for this Example should be prepared fresh on the day of use.

3) Analysis of Samples, Standards, and Blanks:

To each of the prepared solutions, specifically the reagent blanks, N-chloroaniline comparison standard Solution, and the sample solution(s), the following additions should be made in rapid sequence, with thorough mixing between each step:

- 2.5 ml dilute hydrochloric acid solution (20% w/v made by diluting 100 g HCl with 500 ml of water)
- 0.35 ml sodium nitrate solution (10% w/v made by dissolving 1 g of sodium nitrate in 10 ml water
- 2 ml ammonium sulphamate solution (5% w/v or 50 g/l made by dissolving 2.5 g ammonium sulphamate in 50 ml water
- 5 ml NED (1 g/l made by dissolving 0.10 g NED in 100 ml water)
- 1 ml ethanol
- dilute to volume (50 ml) with water.

The mixture is allowed to stand for 30 minutes and then filtered to remove turbidity. The first 2-5 ml filtrate should be discarded. A 20 ml sample of the filtered mixture is placed into a test tube and viewed in ambient light against a sheet of white paper. A visual comparison of any developed red-blue color with the 4-chloroaniline standard solution indicates whether the 4-chloroaniline content of the finished drug product exceeds specifications. If the color developed is equal to or more intense than the standard solution, then the batch does not meet specifications.

_In Vitro_ Release Studies

An _in vitro_ comparative study was conducted using non-compendial grade Sumatra benzoin and Sumatra benzoin BP in the Stage 1 topical dental solution, specifically PREVORA Stage 1, to ascertain the release characteristics of the two versions. Prevora Stage 1, version 1 (Sumatra benzoin) and version 2 (Sumatra benzoin BP), were applied to human teeth which were then submerged in an acid bath (pH 2.2) to simulate demineralization conditions over a 24 hour period. The results are shown graphically in Fig. 11 which is a bar graph of chlorhexidine (CHA) availability as a function of concentration ($\mu$g/ml) _in vitro_ plotted against time (minutes). Referring to Fig. 11, version 1 is the solid bar and version 2 is the striped bar. For example, after 480 minutes, the estimated recovery of chlorhexidine diacetate in the extraction solution is 6% and 39%, respectively, for version 2 and version 1. This indicates that version 2 possessed improved availability of the chlorhexidine on the tooth surface, even under these extreme conditions.

Both versions maintained bactericidal levels of the drug substance over the observation period. Bactericidal levels of chlorhexidine is defined as above 4 $\mu$g/ml, the minium bactericidal concentration reported to be useful against _Streptococcus mutans._ See, for example, Gronroos, et al., Antimicrobial Agents and Chemotherapy, Vol. 39, pages 894-898 (1995); Jardine, et al., European Journal of Oral Sciences, Vol. 103, pages 32-35 (1995). Therefore, both versions have prolonged antimicrobial activity.

Accordingly, it is to be understood that the drawing and description in this disclosure are proffered to facilitate comprehension of the invention, and should not be construed to limit the scope thereof.

**Claims**

1. A method of making a topical, antibacterial coating containing the active ingredient chlorhexidine in ethanolic Sumatra benzoin BP comprising the steps of:

   forming a stock solution of Sumatra benzoin BP in ethanol by mixing Sumatra benzoin BP in ethanol for a period of time just sufficient to dissolve the Sumatra benzoin BP;
   filtering the stock solution sequentially through a series of at least three to five filtration media having successively smaller pore diameter, the smallest pore diameter being 1.2 $\mu$m to form a stock solution having no particulates having a diameter greater than 1.2 $\mu$m;
   mixing chlorhexidine diacetate in the filtered stock solution for a period of time just sufficient to dissolve the chlorhexidine diacetate; and
   adding a quantity of ethanol sufficient to form a finished product of chlorhexidine diacetate in ethanolic Sumatra benzoin BP having the requisite potency of chlorhexidine diacetate to comply with current regulatory requirements.

2. The method of claim 1 wherein the series of filtration media having successively smaller pore diameter is a 2 mm strainer, a 300 $\mu$m filter, a 38 $\mu$m filter, and a 1.2 $\mu$m filter.

3. The method of claim 1 further including the step of applying compressed nitrogen gas to facilitate filtration through the final 1.2 $\mu$m filter.

4. The method of claim 1 wherein the time just sufficient to dissolve the Sumatra benzoin BP is based on peak solubility of the Sumatra benzoin BP in ethanol and the time to dissolve chlorhexidine in the filtered stock solution.

5. The method of claim 1 wherein the finished product of chlorhexidine diacetate in ethanolic Sumatra benzoin BP has a concentration of chlorhexidine of 100.0 to 110.0 mg/ml.

6. A topical, antibacterial coating containing chlorhexidine in Sumatra benzoin BP made by the method of claim 1.

7. The method of claim 1 including the additional steps(s) of testing a sample of the finished drug product for compliance with international regulatory requirements by HPLC.

8. The method of claim 7 wherein a sample of the finished drug product is dissolved in an acidic aqueous medium to precipitate all of the Sumatra benzoin BP from the finished drug product while leaving all of the chlorhexidine diacetate in solution prior to HPLC.

9. The method of claim 8 wherein the acidic aqueous medium comprises 5% phosphoric acid in water.

**10.** The method of claim 9 wherein the step of testing by HPLC comprises determining the concentration (mg/ml) of chlorhexidine diacetate in the finished drug product by HPLC.

**11.** The method of claim 8 including the further step of testing by HPLC comprising determining the presence of degradation products of chlorhexidine diacetate and impurities in the finished drug product.

**12.** The method of claim 11 wherein the degradation products are 4-chloroaniline, 4-chlorophenyl carbodiimide, 4-chlorophenyl isocyanate, 1-[4-chlorophenyl)-5-(6-(3-cyanoguanidino)hexy]biguanide and p-chlorophenyl urea.

**13.** The method of claim 12 wherein 4-chlorophenyl isocyanate is converted to N-4-chlorophenyl ethylcarbamate prior to HPLC.

**14.** The method of claim 1 further including the step of ascertaining the presence of 4-chloroaniline in the finished drug product by a colorimetric test.

**Patentansprüche**

**1.** Verfahren zur Herstellung einer topischen, antibakteriellen Beschichtung, die den Wirkstoff Chlorhexidin in ethanolischem Sumatra-Benzoe BP enthält, das die folgenden Schritte umfasst:

Bilden einer Stammlösung von Sumatra-Benzoe BP in Ethanol durch Mischen von Sumatra-Benzoe BP in Ethanol für eine Zeitdauer, die genau zur Auflösung des Sumatra-Benzoe BP ausreicht;
Filtern der Stammlösung nacheinander durch eine Reihe von mindestens drei bis fünf Filtrationsmedien, die aufeinanderfolgend kleinere Porendurchmesser aufweisen, wobei der kleinste Porendurchmesser 1,2 $\mu$m beträgt, um eine Stammlösung zu bilden, die keine Partikel aufweist, die einen Durchmesser von größer als 1,2 $\mu$m aufweisen;
Mischen von Chlorhexidindiacetat in der gefilterten Stammlösung für eine Zeitdauer, die genau zur Auflösung des Chlorhexidindiacetats ausreicht; und
Zugeben einer Menge an Ethanol, die zur Bildung eines Endprodukts von Chlorhexidindiacetat in ethanolischem Sumatra-Benzoe BP ausreicht, das die erforderliche Wirksamkeit von Chlorhexidindiacetat aufweist, um aktuelle regulatorische Anforderungen zu erfüllen.

**2.** Verfahren nach Anspruch 1, wobei die Reihe von Filtrationsmedien, die aufeinanderfolgend kleinere Porendurchmesser aufweisen, ein Sieb mit 2 mm, ein Filter mit 300 $\mu$m, ein Filter mit 38 $\mu$m und ein Filter mit 1,2 $\mu$m ist.

**3.** Verfahren nach Anspruch 1, das weiter den Schritt des Anwendens eines komprimierten Stickstoffgases einschließt, um eine Filtration durch den letzten Filter mit 1,2 $\mu$m zu ermöglichen.

**4.** Verfahren nach Anspruch 1, wobei die Zeit, die genau zur Auflösung des Sumatra-Benzoe BP ausreicht, auf der höchsten Löslichkeit des Sumatra-Benzoe BP in Ethanol basiert und die Zeit zur Auflösung von Chlorhexidin in der gefilterten Stammlösung.

**5.** Verfahren nach Anspruch 1, wobei das Endprodukt von Chlorhexidindiacetat in ethanolischem Sumatra-Benzoe BP eine Konzentration von Chlorhexidin von 100,0 bis 110,0 mg/ml aufweist.

**6.** Topische, antibakterielle Beschichtung, die Chlorhexidin in Sumatra-Benzoe BP enthält, die durch das Verfahren nach Anspruch 1 hergestellt wird.

**7.** Verfahren nach Anspruch 1, das den/die zusätzlichen Schritt(e) des Prüfens einer Probe des Arzneimittelendprodukts auf die Erfüllung von internationalen regulatorischen Anforderungen durch HPLC einschließt.

**8.** Verfahren nach Anspruch 7, wobei vor der HPLC eine Probe des Arzneimittelendprodukts in einem sauren wässrigen Medium zur Ausfällung des gesamten Sumatra-Benzoe BP aus dem Arzneimittelendprodukt gelöst wird, während das gesamte Chlorhexidinacetat in Lösung verbleibt.

**9.** Verfahren nach Anspruch 8, wobei das saure wässrige Medium 5 % Phosphorsäure in Wasser umfasst.

**10.** Verfahren nach Anspruch 9, wobei der Schritt des Prüfens durch HPLC das Bestimmen der Konzentration (mg/ml) von Chlorhexidindiacetat in dem Arzneimittelendprodukt durch HPLC umfasst.

**11.** Verfahren nach Anspruch 8, das den weiteren Schritt des Prüfens durch HPLC einschließt, der das Bestimmen der Gegenwart von Abbauprodukten von Chlorhexidindiacetat und Verunreinigungen in dem Arzneimittelendprodukt umfasst.

**12.** Verfahren nach Anspruch 11, wobei die Abbauprodukte Folgende sind: 4-Chloranilin, 4-Chlorphenylcarbodiimid, 4-Chlorphenylisocyanat, 1-[4-Chlorphenyl]-5-(6-(3-cyanoguanidino)hexyl]biguanid und p-Chlorphenylharnstoff.

**13.** Verfahren nach Anspruch 12, wobei 4-Chlorphenylisocyanat vor der HPLC in N-4-Chlorphenylethylcarbamat umgewandelt wird.

**14.** Verfahren nach Anspruch 1, das weiter den Schritt des Ermittelns der Gegenwart von 4-Chloranilin in dem Arzneimittelendprodukt durch einen kolorimetrischen Test einschließt.


**Revendications**

**1.** Méthode de préparation d'un revêtement antibactérien topique contenant l'ingrédient actif chlorhexidine dans du benjoin de Sumatra BP éthanolique, comprenant les étapes consistant à :

former une solution stock de benjoin de Sumatra BP dans l'éthanol en mélangeant du benjoin de Sumatra BP dans de l'éthanol pendant une période de temps juste suffisante pour solubiliser le benjoin de Sumatra BP ;
filtrer la solution stock de manière séquentielle à travers une série d'au moins trois à cinq milieux de filtration ayant un diamètre de pores successivement plus petit, le diamètre de pores le plus petit étant de 1,2 $\mu$m, afin de former une solution stock ne contenant aucun matériau particulaire ayant un diamètre supérieur à 1,2 $\mu$m ;
mélanger du diacétate de chlorhexidine dans la solution stock filtrée pendant une période de temps juste suffisante pour solubiliser le diacétate de chlorhexidine ; et
ajouter une quantité d'éthanol suffisante pour former un produit fini de diacétate de chlorhexidine dans du benjoin de Sumatra BP éthanolique ayant la teneur requise en diacétate de chlorhexidine afin de se conformer aux exigences de réglementation actuelles.

**2.** Méthode selon la revendication 1, dans laquelle la série de milieux de filtration ayant un diamètre de pores successivement plus petit est constituée d'une crépine de 2 mm, d'un filtre de 300 $\mu$m, d'un filtre de 38 $\mu$m, et d'un filtre de 1,2 $\mu$m.

**3.** Méthode selon la revendication 1, comprenant en outre l'étape consistant à appliquer de l'azote gazeux comprimé afin de faciliter la filtration à travers le filtre de 1,2 $\mu$m final.

**4.** Méthode selon la revendication 1, dans laquelle le temps juste suffisant pour solubiliser le benjoin de Sumatra BP est basé sur la solubilité maximale du benjoin de Sumatra BP dans l'éthanol et le temps de solubilisation de la chlorhexidine dans la solution stock filtrée.

**5.** Méthode selon la revendication 1, dans laquelle le produit fini de diacétate de chlorhexidine dans du benjoin de Sumatra BP éthanolique possède une concentration en chlorhexidine allant de 100,0 à 110,0 mg/ml.

**6.** Revêtement antibactérien topique contenant de la chlorhexidine dans du benjoin de Sumatra BP, préparé à l'aide de la méthode selon la revendication 1.

**7.** Méthode selon la revendication 1, comportant la ou les étapes supplémentaires consistant à tester un échantillon du produit médicamenteux fini afin de vérifier sa conformité aux exigences de réglementation internationale par HPLC.

**8.** Méthode selon la revendication 7, dans laquelle un échantillon du produit médicamenteux fini est solubilisé dans un milieu aqueux acide afin de précipiter l'ensemble du benjoin de Sumatra BP à partir du produit médicamenteux fini tout en laissant l'ensemble du diacétate de chlorhexidine en solution préalablement à l'HPLC.

**9.** Méthode selon la revendication 8, dans laquelle le milieu aqueux acide comprend 5% d'acide phosphorique dans l'eau.

**10.** Méthode selon la revendication 9, dans laquelle l'étape de test par HPLC comprend la détermination de la concentration (mg/ml) en diacétate de chlorhexidine dans le produit médicamenteux fini par HPLC.

**11.** Méthode selon la revendication 8, comportant l'étape supplémentaire de test par HPLC comprenant la détermination de la présence de produits de dégradation de diacétate de chlorhexidine et d'impuretés dans le produit médicamenteux fini.

**12.** Méthode selon la revendication 11, dans laquelle les produits de dégradation sont la 4-chloroaniline, le 4-chlorophénylcarbodiimide, l'isocyanate de 4-chlorophényle, le 1-[4-chlorophényl)-5-(6-(3-cyanoguanidino)hexyl]biguanide et la p-chlorophényl-urée.

**13.** Méthode selon la revendication 12, dans laquelle l'isocyanate de 4-chlorophényle est converti en N-éthylcarbamate de 4-chlorophényle préalablement à l'HPLC.

**14.** Méthode selon la revendication 1, comportant en outre l'étape consistant à évaluer la présence de 4-chloroaniline dans le produit médicamenteux fini à l'aide d'un test colorimétrique.

# Fig. 1

Dissolution Rate of Sumatra benzoin in ethanol

# *Fig. 2*

Dissolution rate of chlorhexidine acetate in stock solution

## Fig. 3a

DAD1 A, Sig=254,4 Ref=500,100 (CH060804\012-0201.D)

Methanol

## Fig. 3b

DAD1 A, Sig=254,4 Ref=500,100 (CH60808\006-0501.D)

5% $H_3PO_4$/Water

## Fig. 3c

DAD1 A, Sig=254,4 Ref=500,100 (CH60808\007-0601.D)

Stress: 80°C/4days

# Fig. 4a

DAD1 A, Sig=254,4 Ref=500,100 (CH060804\012-0201.D)

Methanol

# Fig. 4b

DAD1 A, Sig=254,4 Ref=500,100 (CH60808\006-0501.D)

5% $H_3PO_4$/Water

# Fig. 4c

DAD1 A, Sig=254,4 Ref=500,100 (CH60808\007-0601.D)

Stress: 80°C/4days

# Fig. 5

## Example Chromatogram of Standard Solution

| SAMPLE INFORMATION | | |
|---|---|---|
| Sample Name: | Std 1 | |
| Sample Type: | Unknown | |
| Vial: | 2 | |
| Injection #: | 1 | |
| Injection Volume: | 10.00 ul | |
| Run Time: | 30.0 Minutes | |
| Sample Set Name: | CHA_2006_09_25 | |

| | Peak Name | RT | Area | Height |
|---|---|---|---|---|
| 1 | CHA | 9.644 | 15553852 | 946634 |

# Fig. 6

## Example Chromatogram of Sample Solution: Prevora Stage 1 Product

| SAMPLE INFORMATION |
| --- |

Sample Name:        Rep 1
Sample Type:        Unknown
Vial:               4
Injection #:        1
Injection Volume:   10.00 ul
Run Time:           30.0 Minutes
Sample Set Name:    CHA_2006_09_25

|   | Peak Name | RT | Area | Height |
|---|-----------|-------|----------|--------|
| 1 |           | 2.931 | 528581   | 42993  |
| 2 |           | 3.203 | 60737    | 7944   |
| 3 |           | 3.832 | 291017   | 44199  |
| 4 |           | 4.330 | 4001514  | 601408 |
| 5 |           | 8.662 | 23872    | 2719   |
| 6 | CHA       | 9.884 | 16302671 | 959924 |

# Fig. 7

## Example Chromatogram of Blank - Diluent (5% phosphoric acid)

SAMPLE   INFORMATION

Sample Name:        Blank-5%H3PO4
Sample Type:        Unknown
Vial:               1
Injection #:        1
Injection Volume:   20.00 ul
Run Time:           40.0 Minutes
Sample Set Name:    CHA_2006_09_14a

| | Peak Name | RT |
|---|---|---|
| 1 | CHA | 18.300 |

# *Fig. 8*

## Example Chromatogram of Standard @ 2.5%

### SAMPLE INFORMATION

Sample Name: Std. 2.5%
Sample Type: Unknown
Vial: 3
Injection #: 3
Injection Volume: 20.00 ul
Run Time: 40.0 Minutes
Sample Set Name: CHA_2006_09_14a

| | Peak Name | RT | Area | Height |
|---|---|---|---|---|
| 1 | | 4.548 | 143663 | 4657 |
| 2 | CHA | 17.912 | 1641899 | 163914 |

# Fig. 9

## Example Chromatogram of Resolution Solution

| SAMPLE INFORMATION | |
|---|---|
| Sample Name: | Resol. Soln. |
| Sample Type: | Unknown |
| Vial: | 2 |
| Injection #: | 1 |
| Injection Volume: | 20.00 ul |
| Run Time: | 40.0 Minutes |
| Sample Set Name: | CHA_2006_09_14a |

| | Peak Name | RT | Area | Height |
|---|---|---|---|---|
| 1 | | 4.161 | 195455 | 7005 |
| 2 | | 6.704 | 110843 | 12300 |
| 3 | | 8.171 | 54544 | 1403 |
| 4 | | 13.447 | 725801 | 60019 |
| 5 | | 16.076 | 529212 | 29956 |
| 6 | | 17.299 | 118642 | 8967 |
| 7 | CHA | 17.925 | 48704284 | 3107931 |
| 8 | | 19.217 | 492261 | 39494 |

28

# Fig. 10

## Example Chromatogram of Prevora Stage 1 Placebo Solution

SAMPLE INFORMATION

Sample Name: Plac unsp (2)
Sample Type: Unknown
Vial: 20
Injection #: 1
Injection Volume: 20.00 ul
Run Time: 40.0 Minutes
Sample Set Name: CHA_2006_09_14a

|   | Peak Name | RT | Area | Height |
|---|-----------|------|---------|--------|
| 1 |           | 3.650 | 32328 | 5678 |
| 2 |           | 3.812 | 125983 | 18824 |
| 3 |           | 4.150 | 173099 | 20812 |
| 4 |           | 4.663 | 41233 | 6332 |
| 5 |           | 6.018 | 178837 | 24182 |
| 6 |           | 6.438 | 33372 | 3997 |
| 7 |           | 6.912 | 72842 | 6478 |
| 8 |           | 8.146 | 6256480 | 518958 |
| 9 |           | 16.759 | 45570 | 4749 |

|    | Peak Name | RT | Area | Height |
|----|-----------|------|------|--------|
| 10 | CHA       | 18.300 |      |        |

## Fig. 11

### CHA Availability Comparison

Bar Graph of the Concentration of Chlorhexidine Acetate
From the *In Vitro* Comparative Study of Two Prevora Products

*Legend:*
    Solid: Proposed Prevora Stage 1 Product I  (lot 46180)
    Striped: Approved Prevora Stage 1 Product II (lot 12020402)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4496322 A

- US 4883534 A

**Non-patent literature cited in the description**

- **T. BALANYK.** Development of sustained-release antimicrobial dental varnishes effective against Streptococcus mutans in vitro. University of Toronto, Faculty of Dentistry, 1986
- chlorhexidine Diacetate. European Pharmacopoeia

- **GRONROOS et al.** *Antimicrobial Agents and Chemotherapy,* 1995, vol. 39, 894-898
- **JARDINE et al.** *European Journal of Oral Sciences,* 1995, vol. 103, 32-35